# EUROPEAN PATENT SPECIFICATION

(11) **EP 1 519 693 B1**
(45) Date of publication and mention of the grant of the patent: **07.04.2010**
(21) Application number: 03761883.2
(22) Date of filing: 18.06.2003
(51) Int. Cl.: A61C 8/00, A61J 1/00, A61J 1/14

(54) **ARRANGEMENT AND METHOD FOR PRESERVING THE NEW-BONEFORMING EFFECT OF GROWTH-STIMULATING SUBSTANCES FOR AN IMPLANT PRODUCT**
ANORDNUNG UND VERFAHREN ZUR KONSERVIERUNG DER NEUE KNOCHEN BILDENDEN WIRKUNG VON WACHSTUMSSTIMULIERENDEN SUBSTANZEN FÜR EIN IMPLANTATIONSPRODUKT
SYSTEME ET PROCEDE DE CONSERVATION DE L'EFFET DE NOUVELLE OSSIFICATION DE SUBSTANCES DE STIMULATION DE LA CROISSANCE D'UN PRODUIT D'IMPLANT

(30) Priority: 01.07.2002 SE 0202052
(43) Date of publication of application: 06.04.2005
(73) Proprietor: Nobel Biocare AB (publ), 402 26 Göteborg (SE)
(72) Inventor: HALL, Jan, S-416 80 Göteborg (SE)
(74) Representative: Byström, Kurt Linus
(86) International application number: PCT/SE2003/001021
(87) International publication number: WO 2004/002357

(56) References cited:
- EP-A2- 0 811 367
- WO-A1-00/72777
- WO-A1-94/03179

## Description

The present invention relates to an arrangement for a container for preserving, even for a long time, the new-bone-forming effect of growth-stimulating substances (GS) applied to one or more implant products. In this connection, the container is arranged so as, dependent on being acted upon, to allow accessibility of the product concerned with applied GS at the time of use of the product. Here, long time means, for example, periods of several years.

It has previously been proposed that a GS which is intended to be used on implants, for example dental implants, should be kept in a frozen environment until use on the spot by a surgeon, dentist etc. The user in question should then mix the frozen molecules with an aqueous solution in order to achieve an appropriate concentration which is to be applied to the product/the implant in question at the point of use. It has also been proposed that the molecules be applied to the product and that the combination of molecules/product be frozen during storage and transport.

Achieving the correct concentration and correct application with the correct application technique on the product in question in order to arrive at a desired result with regard to new-bone growth in the dental context is difficult and requires extensive experience and knowledge. In order to avoid incorrect use *sur place*, it has become desirable for the concentration and application techniques to be performed in the factory and for the product provided with GS to be supplied in finished state to the location where the dental work is to be carried out. In this connection, the need exists to manufacture products with GS in the factory and to effect interim storage of the products in connection with sale and distribution to the users. The object of the present invention is to resolve inter alia this problematic situation and to start from the knowledge the storage time for the GS applied to the product is highly dependent on whether or not the GS is exposed to air, water or a moist environment. In this respect, the greater the exposure of the GS to air, water and moisture, the shorter will be the storage time in order for effective use of the product in question with GS to be possible. The invention also aims to solve this problem by making possible long storage times in this connection, that is to say storage times of several years.

The invention is defined by independent claims 1 and 6.

An arrangement comprises a container enclosing the product or the products with applied GS in an environment which is essentially free from air, water and moisture.

In this connection, the container can be in the form of a glass ampoule, or be made of metal which makes the environment free from air, water and moisture possible, for example metal in the form of titanium, stainless steel etc. The container can consist of a vacuum container which has an internal pressure for the product or the products with applied GS of <10⁻⁴ mbar. Said environment can comprise one or more essentially inert gases, for example argon, nitrogen or helium, which in one embodiment are used in connection with evacuation of the container in question.

A method comprises enclosing in the container the product or the products with applied GS in an environment which is essentially free from air, water and moisture. In one embodiment, the product with associated GS can be applied in a glass tube provided with a bottom, the interior of which is connected to a vacuum pump. After evacuation, a burning means employed can be activated for formation of a closed glass ampoule by sealing the glass tube part enclosing the product with GS. In the case of metal, a first part or lower part made of foil-shaped metal, for example titanium or stainless steel, can be used. The product is arranged on the first part with associated GS applied. A second part or upper part likewise made of foil-shaped metal is applied to the first part or lower part with the product or the products enclosed. The space between the parts which is intended for the product or the products is evacuated and/or filled with gas and sealed by means of laser welding or what may be referred to as extended single-spot welding, that is to say welding by means of one stroke along the entire sealing length. In some applications, seaming can also be employed as the sealing method. Other developments of the method emerge from the subsequent subclaims for the method.

By virtue of what is proposed above, accurate production of optimum products loaded or provided with GS can take place in the factory. Even after a long storage time, the product with GS can give optimum results with regard to new-bone growth in various dental situations. As examples of GS, mention may be made of matrix molecules, growth and differentiation factors and peptides with growth-stimulating properties. GS can also be applied to substances such as autologous bone, allogenic bone or synthetic preparations, these substances and preparations also then having a long storage time with the principle employed. When air containing water and moisture is replaced by inert gas free from water or moisture or evacuation of the container concerned is effected, known equipment can be used for the gas supply and evacuation.

A for the present proposed arrangement and method based on the characteristics significant of the invention will be described below with simultaneous reference to accompanying drawings in which
- Figure 1: shows in the form of a schematic diagram the formation of a glass ampoule with a product provided with GS introduced, the interior of the glass ampoule being connectable to on the one hand a vacuum pump and on the other hand a gas bottle;
- Figure 1a: shows in principle the sealing function for the glass ampoule;
- Figure 1b: shows in principle a pumping arrangement for a number of ampoules;
- Figure 2: shows a horizontal view of a first part or lower part made of metal foil with a compartment for an implant or another product with applied GS;
- Figure 3: shows a vertical section of the first part or lower part according to Figure 2;
- Figure 4: shows a horizontal view from above of a second part or upper part intended to be applied to the first part or lower part and provided with a tear-off strip for uncovering the product with applied GS enclosed by the first and second parts;
- Figure 5: shows a side view of the upper part according to Figure 4;
- Figure 6: shows a horizontal view of the first and second parts in a joined-together state, the parts having been joined together by means of a welding procedure, and
- Figure 7: shows a side view of a part of the welding tool for extended single-spot welding which brings about the mutual sealing of the first and second parts with the implant with GS located inside.

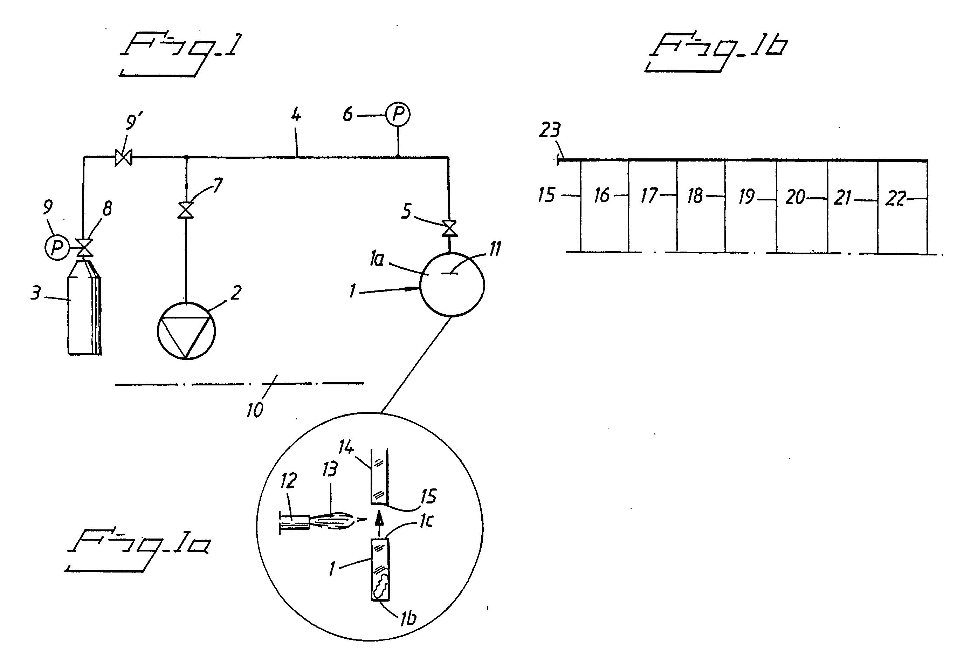

In Figure 1, what will become a glass ampoule is indicated symbolically by 1. A vacuum pump of a kind known per se is shown by 2 and a gas container by 3. The ampoule is connected to a line system 4 which is indicated schematically. The connection of the interior 1a of the glass ampoule to the line system is effected by means of melting the glass material. Also connected to the system is a manometer 6 in order to make it possible to establish an appropriate pressure in the system. The pump 2 is connected to the line system via valve means 7. The gas bottle 3 is provided with a regulating valve 8, to which a manometer, here called the second manometer 9, can be connected. The gas bottle arrangement can be connected to the line system 4 via a second valve means 9'. A product indicated schematically, for example an implant, which has the reference number 11, is introduced into the glass ampoule. The product is provided with GS in the manner indicated in the applications filed by the same applicant and inventor. In this connection, reference may be made to patent applications WO 00/72775 and WO 00/72777 by the same applicant as in the present patent application. Reference may also be made to the article "Properties of a New Porous Oxide Surface on Titanium Implants, Volume 1: The Oxidized Titanium Surface, Applied Osseointegration Research", October 2000, published by among others the inventor according to the present patent application.

A technique known per se can be used for sealing the ampoule 1. By means of the vacuum pump, the interior of the ampoule is evacuated to a value which is <10⁻⁴ in the illustrative embodiment. In the space 10, the glass ampoule thus evacuated is sealed with the aid of a rotating burner 12 according to Figure 1. The burner works with a gas flame 13. The glass ampoule 1 is provided with a sealed bottom part 1b, and the top part 1c is also sealed by means of the gas flame 13. As the glass ampoule is provided with an indication, it can then be opened by breaking. In some cases, it is desirable for the glass ampoule to be provided with a positive pressure by an inert gas, for example argon, in order to prevent glass fragments entering the ampoule when it is broken. The ampoule is then filled with gas from the gas bottle 3 which is connected via the regulating valve 8 and the valve means 9 to the line system and via the connection valve 5.

Figure 1 shows a multiple arrangement for a number of glass ampoules. Each glass ampoule 15, 16, 17, 18, 19, 20, 21 and 22 is connected internally to a line 23, which can in turn be connected in a manner known per se to said gas bottle 3 and the vacuum pump. Products provided with GS are introduced into the ampoules, after which the latter can be evacuated, filled with gas and sealed in accordance with the above.

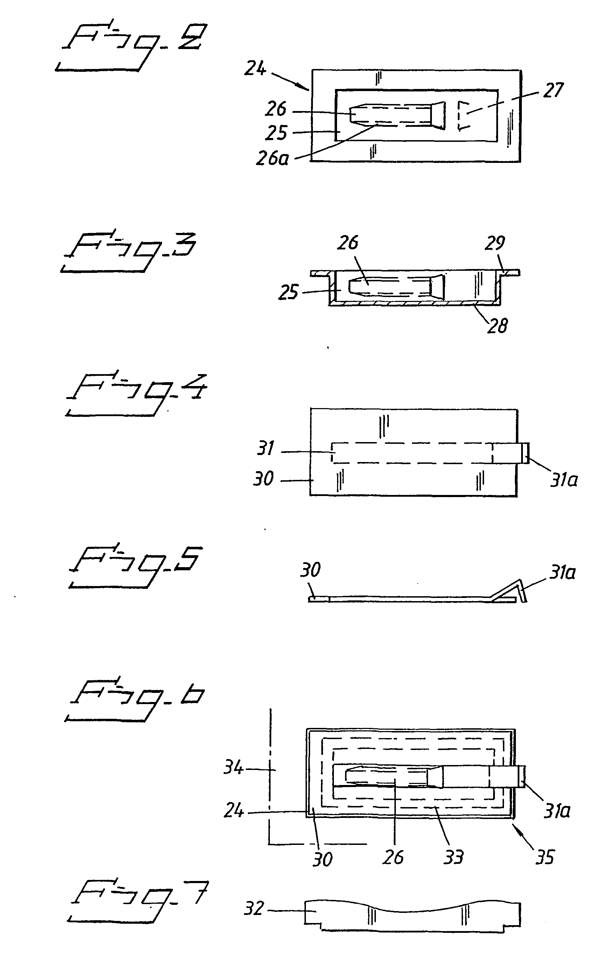

Figures 2-7 show the case when the container consists of two foil-shaped metal parts which can be welded together with one another with the product with GS located inside during simultaneous evacuation of the space for the product in connection with evacuation. Figure 2 shows a first part or lower part 24 with a space 25 for a product in the form of an implant 26 which has been coated with GS on its external thread 26a in the way indicated above. The space 25 can contain more than one product, which has been symbolized by 27.

Figure 3 shows the shape of the first part or lower part from the side; the part can be regarded as comprising a space part 28 and a flange part 29.

Figure 4 shows a second part or upper part which is intended to be joined together with the part according to Figures 2 and 3. The second part or upper part is indicated by 30 and is provided with a tear-off strip 31. The second part or upper part is arranged in a vacuum-tight manner in relation to the lower part by means of welding around the flange part, for example laser welding. The tear-off strip has at one of its ends a tab 31a, by means of which it is possible to take hold of the strip and tear the same off from the part 30 in question.

In accordance with Figure 5, the tab part 31a has a shape which is easy to grip and facilitates tearing-off of the flap 31 from the part 30.

Figure 6 shows the case where the second part 30 has been applied to the first part 24. Sealing is effected by means of laser welding or the welding stamp 32 shown symbolically in Figure 7 which is arranged to establish a vacuum-tight seal 33 between the parts 24 and 30 by interaction. Sealing is effected at the flange 29 shown in Figure 3. Sealing is effected by a single interaction between the stamp and the parts 30 and 40, and the welding function is referred to here as extended single-spot welding. Stamping is preceded by evacuation and in some cases by a subsequent gas-filling of the inner space (compare 25 in Figure 3) of the parts, which evacuation and gas-filling are performed in a symbolically indicated apparatus 34 of a type known per se. Reference is also made to the arrangement above according to Figures 1-1b. The weld 33 runs all the way round and on the whole follows the center line of the flange 29. The container 35 thus evacuated makes possible long-term storage of the product 26 with applied GS for said long time without the GS degenerating with regard to its new-bone-forming function.

## Claims

1. An arrangement for preserving, even for a long time comprising a container (35) and one or more implant products, for example in the form of dental implants, with applied growth-stimulating substances (26a) the container being arranged so as, dependent on being acted upon, to allow accessibility of the product concerned with applied growth-stimulating substances at the time of its use, wherein the container (35) encloses the product or the products with applied growth-stimulating substances (26a) in an environment which is essentially free from air, water and moisture.

2. The arrangement as claimed in claim 1, **characterized in that** the container is in the form of a glass ampoule.

3. The arrangement as claimed in claim 1, **characterized in that** the container is made of metal which makes the environment free from air, water and moisture possible, for example titanium, stainless steel etc.

4. The arrangement as claimed in claim 1, 2 or 3, **characterized in that** container has been evacuated to an internal pressure for the product or the products with applied growth-stimulating substances of <1 mbar, preferably <10⁻³ mbar..

5. The arrangement as claimed in claim 1,2 or 3, **characterized in that** said environment comprises one or more essentially inert gases free from air, water and moisture, for example argon.

6. A method for enclosing one or more products for preserving, even for a long time, for example in the form of or comprising implants with applied growth-stimulating substances in a container (35), the container (35) being arranged to be openable dependent on being acted upon so as to allow accessibility of the product with applied growth-stimulating substances (26a) at the time of use of the product, wherein by enclosing the product or the products with applied growth-stimulating substances (26a) in the container (35) in an environment which is essentially free from air, water and moisture.

7. The method as claimed in claim 6, **characterized in that** the container is made as a glass ampoule or of metal capable of preserving the environment free from air, water and moisture, for example titanium, stainless steel etc.

8. The method as claimed in claim 6 or 7, **characterized in that** the container is evacuated with an internal pressure of <1 mbar, preferably <10⁻³ mbar.

9. The method as claimed in claim 6 or 7, **characterized in that** the environment free from air, water and moisture is formed by means of one or more gases free from water and moisture, for example argon.

10. The method as claimed in any one of claims 6-9, **characterized in that** the product concerned with associated growth-stimulating substances is introduced into an open glass ampoule, the interior of which is connected to a vacuum pump, and **in that**, when a low internal vacuum pressure has been reached, the glass ampoule is sealed using a burning means, for example a rotating burner.

11. The method as claimed in claim 10, **characterized in that** the product concerned with associated growth-stimulating substances is introduced into a glass tube provided with a bottom, the interior of which is connected to the vacuum pump, and **in that** the burning means is activated for formation of a closed glass ampoule by sealing the glass tube part enclosing the product with GS.

12. The method as claimed in claim 10 or 11, **characterized in that** the interior of the ampoule or of the glass tube part is connected temporarily to a gas container, for example an argon gas container.

13. The method as claimed in any one of claims 6-9, **characterized in that** the product or the products with applied growth-stimulating substances is or are arranged in a first part or lower part made of foil-shaped metal, for example titanium or stainless steel, **in that** a second part or upper part likewise made of foil-shaped metal is applied to the first part or lower part and over the product or the products, **in that** a space between the first and second parts or, respectively, the lower and upper parts which is intended for the product or the products with applied growth-stimulating substances is evacuated and/or filled with gas and sealed by means of extended single-spot welding or laser welding.

14. The method as claimed in claim 13, **characterized in that** the first part or lower part and/or the second part or upper part is/are made with a tear-off foil strip for access to the product/the products with growth-stimulating substances at the time of use.

## Patentansprüche

1. Anordnung zum Konservieren, auch für eine lange Zeit, die einen Behälter (35) und ein oder mehrere Implantatprodukte, zum Beispiel in der Form von Zahnimplantaten, mit applizierten wachstumsstimulierenden Substanzen (26a) umfasst, wobei der Behälter so eingerichtet ist, dass er, abhängig von Einwirkung darauf, den Zugriff auf das betreffende Produkt mit applizierten wachstumsstimulierenden Substanzen zum Zeitpunkt seiner Verwendung erlaubt, wobei der Behälter (35) das Produkt oder die Produkte mit applizierten wachstumsstimulierenden Substanzen (26a) in einer Umgebung einschließt, die im Wesentlichen frei von Luft, Wasser und Feuchtigkeit ist.

2. Anordnung gemäß Anspruch 1, **dadurch gekennzeichnet, dass** der Behälter in Form einer Glasampulle vorliegt.

3. Anordnung gemäß Anspruch 1, **dadurch gekennzeichnet, dass** der Behälter aus Metall hergestellt ist, das die Umgebung frei von Luft, Wasser und Feuchtigkeit möglich macht, zum Beispiel Titan, rostfreier Stahl, usw.

4. Anordnung gemäß Anspruch 1, 2 oder 3, **dadurch gekennzeichnet, dass** der Behälter auf einen Innendruck für das Produkt oder die Produkte mit applizierten wachstumsstimulierenden Substanzen von <1 mbar, bevorzugt < 10⁻³ mbar evakuiert ist.

5. Anordnung gemäß Anspruch 1, 2 oder 3, **dadurch gekennzeichnet, dass** die Umgebung ein oder mehrere im Wesentlichen inerte Gase frei von Luft, Wasser und Feuchtigkeit, zum Beispiel Argon, umfasst.

6. Verfahren zum Einschließen eines oder mehrerer Produkte zum Konservieren, auch für einen langen Zeitraum, zum Beispiel in der Form von oder umfassend Implantate mit applizierten wachstumsstlmulierenden Substanzen in einem Behälter (35), wobei der Behälter (35) eingerichtet ist, abhängig von Einwirkung darauf, öffnenbar zu sein, um so die Zugänglichkeit des Produkts mit applizierten wachstumsstimulierenden Substanzen (26a) zum Zeitpunkt der Verwendung des Produkts zu ermöglichen, wobei das Produkt oder die Produkte mit applizierten wachstumsstimulierenden Substanzen (26a) in dem Behälter (35) in einer Umgebung eingeschlossen ist, die im Wesentlichen frei von Luft, Wasser und Feuchtigkeit ist.

7. Verfahren gemäß Anspruch 6, **dadurch gekennzeichnet, dass** der Behälter als eine Glasampulle oder aus Metall, das fähig ist, die Umgebung frei von Luft, Wasser und Feuchtigkeit zu konservieren, zum Beispiel Titan, rostfreier Stahl, usw., hergestellt ist.

8. Verfahren gemäß Anspruch 6 oder 7, **dadurch gekennzeichnet, dass** der Behälter mit einem Innendruck von < 1 mbar, bevorzugt < 10⁻³ mbar evakuiert ist.

9. Verfahren gemäß Anspruch 6 oder 7, **dadurch gekennzeichnet, dass** die Umgebung frei von Luft, Wasser und Feuchtigkeit mittels eines oder mehrerer Gase frei von Wasser und Feuchtigkeit, zum Beispiel Argon, gebildet ist.

10. Verfahren gemäß einem der Ansprüche 6 bis 9, **dadurch gekennzeichnet, dass** das betreffende Produkt mit assoziierten wachstumsstimulierenden Substanzen in eine offene Glasampulle eingeführt wird, deren Inneres mit einer Vakuumpumpe verbunden ist, und dass, wenn ein niedriger innerer Vakuumdruck erreicht worden ist, die Glasampulle unter Verwendung eines Heizmittels, zum Beispiel eines rotierenden Brenners, versiegelt wird.

11. Verfahren gemäß Anspruch 10, **dadurch gekennzeichnet, dass** das betreffende Produkt mit assoziierten wachstumsstimulierenden Substanzen in ein Glasrohr, das mit einem Boden versehen ist, eingeführt wird dessen Inneres mit der Vakuumpumpe verbunden ist, und dass das Heizmittel zur Bildung einer geschlossenen Glasampulle durch Versiegeln des Glasrohrteils, der das Produkt mit WS einschließt, aktiviert wird.

12. Verfahren gemäß Anspruch 10 oder 11, **dadurch gekennzeichnet, dass** das Innere der Ampulle oder des Glasröhrenteils temporär mit einem Gasbehälter, zum Beispiel einem Argongasbehälter, verbunden wird.

13. Verfahren gemäß einem der Ansprüche 6 bis 9, **dadurch gekennzeichnet, dass** das Produkt oder die Produkte mit applizierten wachstumsstimulierenden Substanzen in einem ersten Teil oder einem unteren Teil, der aus folienförmigem Material, zum Beispiel Titan oder rostfreiem Stahl, hergestellt ist, angeordnet ist oder sind, dass ein zweiter Teil oder oberer Teil, der ebenfalls aus folienförmigem Material hergestellt ist, auf den ersten Teil oder unteren Teil und über dem Produkt oder den Produkten aufgebracht wird, dass ein Raum zwischen den ersten und den zweiten Teilen oder jeweils den unteren und oberen Teilen, der für das Produkt oder die Produkte mit applizierten wachstumsstimulierenden Substanzen vorgesehen ist, evakuiert und/oder mit Gas gefüllt und mittels ausgedehntem Einzelpunktschweißen oder Laserschweißen versiegelt wird.

14. Verfahren gemäß Anspruch 13, **dadurch gekennzeichnet, dass** der erste Teil oder untere Teil und/oder der zweite Teil oder obere Teil mit einem Abreiß-Folienstreifen zum Zugriff auf das Produkt/die Produkte mit wachstumsstimulierenden Substanzen zum Zeitpunkt der Verwendung hergestellt wird/werden.

## Revendications

1. Système pour la conservation, même pendant une longue période de temps, comprenant un récipient (35) et un ou plusieurs produits d'implant, par exemple se présentant sous la forme d'implants dentaires, avec des substances de stimulation de croissance appliquées (26a), le récipient étant agencé afin de, en fonction du fait qu'il est actionné, permettre l'accessibilité du produit concerné avec les substances de stimulation de croissance appliquées au moment de son utilisation, dans lequel le récipient (35) enferme le produit ou les produits avec les substances de stimulation de croissance appliquées (26a) dans un environnement qui est essentiellement dépourvu d'air, d'eau et d'humidité.

2. Système selon la revendication 1, **caractérisé en ce que** le récipient se présente sous la forme d'une ampoule en verre.

3. Système selon la revendication 1, **caractérisé en ce que** le récipient est réalisé à partir de métal qui permet de débarrasser l'environnement d'air, d'eau et d'humidité, par exemple le titane, l'acier inoxydable, etc.

4. Système selon la revendication 1, 2 ou 3, **caractérisé en ce que** le récipient a été mis sous vide à une pression interne pour le produit ou les produits avec les substances de stimulation de croissance appliquées de < 1 mbar, de préférence < 10⁻³ mbar.

5. Système selon la revendication 1, 2 ou 3, **caractérisé en ce que** ledit environnement comprend un ou plusieurs gaz essentiellement inertes dépourvus d'air, d'eau et d'humidité, par exemple l'argon.

6. Procédé pour enfermer un ou plusieurs produits pour la conservation, même pendant une longue période de temps, par exemple se présentant sous la forme de ou comprenant des implants avec des substances de stimulation de croissance appliquées dans un récipient (35), le récipient (35) étant agencé pour être ouvert en fonction de son actionnement, pour permettre l'accessibilité du produit avec les substances de stimulation de croissance appliquées (26a), au moment de l'utilisation du produit, dans lequel on enferme le produit ou les produits avec les substances de stimulation de croissance appliquées (26a) dans le récipient (35) dans un environnement qui est essentiellement dépourvu d'air, d'eau et d'humidité.

7. Procédé selon la revendication 6, **caractérisé en ce que** le récipient est réalisé sous la forme d'une ampoule en verre ou en métal pouvant conserver l'environnement dépourvu d'air, d'eau et d'humidité, par exemple le titane, l'acier inoxydable, etc.

8. Procédé selon la revendication 6 ou 7, **caractérisé en ce que** le récipient est mis sous vide avec une pression interne de < 1 mbar, de préférence < 10⁻³ mbar.

9. Procédé selon la revendication 6 ou 7, **caractérisé en ce que** l'environnement dépourvu d'air, d'eau et d'humidité est formé au moyen d'un ou de plusieurs gaz dépourvus d'eau et d'humidité, par exemple l'argon.

10. Procédé selon l'une quelconque des revendications 6 à 9, **caractérisé en ce que** le produit concerné avec les substances de stimulation de croissance associées, est introduit dans une ampoule en verre ouverte, dont l'intérieur est raccordé à une pompe de vide, et **en ce que**, lorsque l'on a atteint une faible pression de vide interne, l'ampoule en verre est hermétiquement fermée en utilisant des moyens de combustion, par exemple un brûleur rotatif.

11. Procédé selon la revendication 10, **caractérisé en ce que** le produit concerné avec des substances de stimulation de croissance associées est introduit dans un tube en verre prévu avec un fond, dont l'intérieur est raccordé à la pompe de vide, et **en ce que** les moyens de combustion sont activés pour la formation d'une ampoule en verre fermée en fermant hermétiquement la partie de tube en verre enfermant le produit avec du GS.

12. Procédé selon la revendication 10 ou 11, **caractérisé en ce que** l'intérieur de l'ampoule ou de la partie de tube en verre est raccordé temporairement à un récipient de gaz, par exemple un récipient d'argon.

13. Procédé selon l'une quelconque des revendications 6 à 9, **caractérisé en ce que** le produit ou les produits avec les substances de stimulation de croissance appliquées est ou sont agencés dans une première partie ou partie inférieure réalisée à partir d'un métal en forme de feuille, par exemple du titane ou de l'acier inoxydable, **en ce qu'**une seconde partie ou partie supérieure également réalisée à partir d'un métal en forme de feuille est appliquée sur la première partie ou partie inférieure et sur le produit ou les produits, **en ce qu'**un espace entre les première et seconde parties ou bien, respectivement les parties inférieure et supérieure, qui est prévu pour le produit ou les produits avec des substances de stimulation de croissance appliquées est mis sous vide et/ou rempli avec un gaz et fermé hermétiquement au moyen d'un soudage par points unique étendu ou d'un soudage au laser.

14. Procédé selon la revendication 13, **caractérisé en ce que** la première partie ou partie inférieure et/ou la seconde partie ou partie supérieure est / sont réalisée(s) avec une bande de feuille déchirable pour avoir accès au produit / aux produits avec les substances de stimulation de croissance au moment de l'utilisation.
